# EUROPEAN PATENT SPECIFICATION

(11) **EP 3 500 115 B1**
(45) Date of publication and mention of the grant of the patent: **30.09.2020**
(21) Application number: 17749485.3
(22) Date of filing: 10.08.2017
(51) Int. Cl.: A24D 1/02, A24F 47/00

(54) **AEROSOL-GENERATING ARTICLE HAVING IMPROVED WRAPPER**
AEROSOLERZEUGUNGSARTIKEL MIT VERBESSERTER VERPACKUNG
ARTICLE DE GÉNÉRATION D'AÉROSOL AYANT UN EMBALLAGE AMÉLIORÉ

(30) Priority: 17.08.2016 EP 16184601
(43) Date of publication of application: 26.06.2019
(73) Proprietor: Philip Morris Products S.A., 2000 Neuchâtel (CH)
(72) Inventor: LANG, Gerhard, 3280 Murten (CH); MALGAT, Alexandre, 1422 Les Tuileries de Granson (CH); VUARNOZ-BIZE, Aline, 1745 Lentigny (CH)
(74) Representative: Nytko-Lutz, Emily Anne
(86) International application number: PCT/EP2017/070381
(87) International publication number: WO 2018/033477

(56) References cited:
- EP-B1- 2 077 731
- WO-A2-2011/117750
- US-A- 4 015 610
- US-A1- 2005 016 549
- US-A1- 2005 121 047
- US-A1- 2005 263 163
- US-A1- 2007 095 358
- US-A1- 2016 066 618
- US-A1- 2016 205 996
- US-B1- 6 615 840

## Description

The present invention relates to a novel wrapper for a heat-not-burn article and to a heat-not-burn article incorporating such a wrapper.

A number of smoking articles in which tobacco is heated rather than combusted have been proposed in the art. One aim of such heated smoking articles is to reduce known harmful smoke constituents of the type produced by the combustion and pyrolytic degradation of tobacco in conventional cigarettes.

Typically in such heated smoking articles, an aerosol is generated by the transfer of heat from a heat source to a physically separate aerosol-forming substrate or material, which may be located within, around or downstream of the heat source. During smoking, volatile compounds are released from the aerosol-forming substrate by heat transfer from the heat source and entrained in air drawn through the smoking article. As the released compounds cool, they condense to form an aerosol that is inhaled by the user.

A number of prior art documents disclose aerosol-generating devices for consuming or smoking heated smoking articles. Such devices include, for example, electrically heated aerosol-generating devices in which an aerosol is generated by the transfer of heat from one or more electrical heating elements of the aerosol-generating device to the aerosol-forming substrate of a heated smoking article. One advantage of such electrical smoking systems is that they significantly reduce sidestream smoke, while permitting a user to selectively suspend and reinitiate smoking.

During the use of electrically heated aerosol-generating devices, the power supplied to the heating element is controlled in order to achieve a specific heating profile that provides a substantially consistent aerosol delivery to the consumer over time. During a first phase of the heating profile, referred to herein as the "pre-heating phase", power is provided to the heating element to raise it to from the ambient temperature to a first temperature, at which aerosol is generated from the aerosol-forming substrate. In many devices, it is desirable to generate aerosol with the desired constituents as soon as possible after activation of the device, since consumers do not want to have to wait for a significant period following activation of the device before having a first puff. For this reason, in the first phase, power may be supplied to the heating element to raise it to the first temperature as quickly as possible. Following the pre-heating phase, the heating profile shifts to a second heating phase in which power is supplied to the heating element to retain it at a second temperature, typically lower than the first temperature, to achieve a consistent delivery of aerosol to the consumer as the consumer puffs on the heat-not-burn article.

It has been found that during the pre-heating phase, certain compounds are released from the aerosol-forming substrate as it heats up, which produce an undesirable malodour that may be detected by the consumer. One of the main compounds causing this malodour has been identified as hydrogen sulphide, which has an unpleasant sulphurous odour. Other compounds such as methanethiol and carbonyl sulphide may also contribute to the malodour, although typically to a lesser extent.

US 4 015 610 A relates to a cigarette filter tip comprising a central element comprising granular iron sulphate. The granular iron sulphate in the tip changes colour upon reaction with hydrogen sulphide in the cigarette smoke to form an iron sulphide.

It would be desirable to provide a heat-not-burn article with novel means for reducing malodour during the pre-heating phase. It would be particularly desirable to provide a heat-not-burn article having means for reducing malodour that can be incorporated without significant modification of the construction of the heat-not-burn article. It would further be desirable to provide such means for reducing malodour that can be incorporated with minimal impact on the smoking experience for the consumer.

According to a first aspect of the invention there is provided a wrapper for a heat-not-burn article comprising an aerosol-forming tobacco substrate, the wrapper comprising a sulphide scavenger compound, wherein the sulphide scavenger compound is a metal salt, wherein the metal salt is a carbonate, chloride, hydroxide, nitrate, malate, acetate, citrate or bromide, and wherein the sulphide scavenger compound is based on a transition metal.

According to a second aspect of the invention there is provided a heat-not-burn article comprising: an aerosol-forming tobacco substrate; and a wrapper according to the first aspect of the invention circumscribing at least a part of the heat-not-burn article.

Preferably, the heat-not-burn article is an aerosol-generating article for use in an aerosol-generating device comprising a heating element. However, in other embodiments the heat-not-burn article may itself incorporate a heat source, such as a carbon heat source, for heating the aerosol-forming substrate.

According to a third aspect of the invention there is provided an aerosol-generating system comprising: an aerosol-generating device comprising a heating element; and a heat-not-burn article for use with the aerosol-generating device, the heat-not-burn article comprising: an aerosol-forming tobacco substrate; and a wrapper according to the first aspect of the invention circumscribing at least a part of the heat-not-burn article.

According to a fourth aspect of the invention there is provided a method for the production of a heat-not-burn article, the method comprising the steps of: providing an aerosol-forming tobacco substrate; combining the aerosol-forming tobacco substrate with one or more components to form the heat-not-burn article; and wrapping the heat-not-burn article with an outer wrapper, wherein the outer wrapper is a wrapper according to the first aspect of the invention.

According to a fifthth aspect of the invention there is provided a use of a sulphide scavenger compound based on a transition metal salt in the wrapper of a heat-not-burn article to reduce the level of hydrogen sulphide released during heating of the heat-not-burn article.

Features described below in relation to one aspect or embodiment of the invention may also be applicable to other aspects and embodiments. For example, features described in relation to the wrapper of heat-not-burn articles according to the invention will typically also be applicable to the wrapper of the heat-not-burn articles of aerosol-generating systems according to the invention and to wrappers according to the invention.

As used herein, the term "heated aerosol-generating article" refers to a heat-not-burn article comprising an aerosol-forming substrate that, when heated, releases volatile compounds that can form an aerosol. The aerosols generated from aerosol-forming substrates of smoking articles according to the invention may be visible or invisible and may include vapours (for example, fine particles of substances, which are in a gaseous state, that are ordinarily liquid or solid at room temperature) as well as gases and liquid droplets of condensed vapours.

As used herein, the term "aerosol-generating device" refers to a device that interacts with an aerosol-forming substrate of an heat-not-burn article to generate an aerosol.

As used herein, the terms "upstream" and "downstream" are used to describe the relative positions of elements, or portions of elements, of the heat-not-burn article in relation to the direction in which a user draws on the heat-not-burn article during use thereof.

As used herein, the term "sulphide scavenger compound" refers to a compound that has the potential to chemically react with sulphide compounds such as hydrogen sulphide to convert them into a less volatile form. Certain sulphide scavenger compounds may additionally act to reduce other sulphur compounds, including for example mercaptans, such as methanethiol.

Heat-not-burn articles according to the present invention incorporate a sulphide scavenger compound into the wrapper which acts to remove at least a proportion of the hydrogen sulphide released from the aerosol-forming substrate during the pre-heating phase described above. The hydrogen sulphide is thereby prevented from reaching the consumer such that the malodour during pre-heating can be effectively reduced or eliminated.

By providing the sulphide scavenger compound in a wrapper circumscribing the heat-not-burn article, the sulphide scavenger compound is advantageously positioned to come into contact with the sulphide compounds that are released from the aerosol-forming substrate during pre-heating.

The sulphide scavenger compound can advantageously be incorporated into the wrapper prior to the assembly of the heat-not-burn article so that the construction and manufacture of the heat-not-burn article are substantially unaffected. The incorporation of the sulphide scavenger compound into the wrapper means that the impact on the composition of the aerosol delivered to the consumer during use is minimised.

In heat-not-burn articles according to the invention, the sulphide scavenger compound is preferably incorporated into the outer wrapper circumscribing the heat-not-burn article. Alternatively or in addition, the sulphide scavenger compound may be incorporated into one or more plug wraps circumscribing the components of the heat-not-burn article beneath the outer wrapper. Preferably, where the sulphide scavenger compound is incorporated into one or more plug wraps, the sulphide scavenger compound is incorporated into at least the plug wrap circumscribing the aerosol-forming substrate.

As defined above, the wrapper of heat-not-burn articles according to the invention incorporates a sulphide scavenger compound that is metal-based. This means that the compound comprises a metal as one of the main constituents. The wrapper comprises a sulphide scavenger compound that is a metal salt.The sulphide scavenger compound is based on a transition metal. The term "transition metal" is used herein to refer to a metal in the d-block of the Periodic table. Preferably, the transition metal is selected from the group consisting of zinc, iron and copper. In particularly preferred embodiments, the sulphide scavenger compound is based on zinc.The sulphide scavenger compound is a transition metal salt., The metal salt is a carbonate, chloride, sulphate, hydroxide, nitrate, malate, acetate, citrate or bromide.

Suitable zinc based sulphide scavenger compounds include but are not limited to: zinc carbonate, basic zinc carbonate, zinc chloride, zinc sulphate, zinc acetate and zinc bromide.

Suitable iron based sulphide scavenger compounds include but are not limited to: iron sulphate.

Suitable copper based sulphide scavenger compounds include but are not limited to: cupric carbonate, cupric sulphate, cupric nitrate and cupric chloride.

The sulphide scavenger compound may be incorporated into or onto the wrapper in a variety of different ways. The sulphide scavenger compound may be incorporated directly into or onto the wrapper, for example, in the form of a solid powder. Alternatively, the sulphide scavenger compound may be combined with a suitable binder, for example, a polymeric binder. This may facilitate the application of the sulphide scavenger compound into or onto the wrapper. Suitable polymeric binders include but are not limited to PVA and cellulosic binders.

In certain embodiments, the sulphide scavenger compound is incorporated in an outer layer provided on at least one surface of the wrapper. For example, the wrapper may be coated by an outer layer of the sulphide scavenger compound provided on the inside or outside or both the inside and outside of the wrapper with respect to the aerosol-forming substrate. The outer layer is preferably applied to the surface or surfaces of the wrapper in the form of a solution incorporating the sulphide scavenger compound. The sulphide scavenger compound may be applied to the wrapper together with a polymeric binder, as described above.

In alternative embodiments, the wrapper is formed of a sheet of fibrous material such as paper, wherein the sulphide scavenger compound is dispersed within the fibrous material. In such embodiments, the sulphide scavenger compound is typically added as a filler during production of the sheet of fibrous material. For example, where the sheet of fibrous material is paper, the sulphide scavenger compound can be added into the pulp during the paper-making process.

In further alternative embodiments, the sulphide scavenger compound is impregnated into the wrapper. In such embodiments, the sulphide scavenger compound is impregnated into the structure of the wrapper, in contrast to a coating layer as described above, in which the sulphide scavenger compound is provided in a layer on the surface of the wrapper. The impregnation of the sulphide scavenger compound is typically achieved by pressing to incorporate the sulphide scavenger compound into an existing wrapper, for example, using one or more rollers. The sulphide scavenger compound will typically be impregnated in the form of a solution.

The wrapper preferably incorporates at least about 0.01 percent by weight of the metal component of the sulphide scavenger compound, more preferably at least about 0.1 percent by weight, more preferably at least about 0.25 percent by weight based on the total combined weight of the wrapper and the sulphide scavenger compound. This effectively corresponds to the "concentration" by weight of the metal component in the wrapper. Alternatively or in addition, the wrapper preferably incorporates no more than about 5 percent by weight of the metal component of the sulphide scavenger compound, more preferably no more than about 4 percent by weight, based on the total combined weight of the wrapper and the sulphide scavenger compound. Preferably the wrapper incorporates between about 0.01 percent and about 5 percent by weight of the metal component of the sulphide scavenger compound, more preferably between about 0.1 percent and about 4 percent by weight, more preferably between about 0.25 percent and about 4 percent by weight, based on the total combined weight of the wrapper and the sulphide scavenger compound.

The wrapper preferably incorporates at least about 0.25 micrograms of the metal component of the sulphide scavenger compound per square centimetre of the wrapper, preferably at least about 2.5 micrograms per square centimetre of the wrapper, more preferably at least about 5 micrograms per square centimetre over the area of the wrapper into which the sulphide scavenger compound is incorporated. Alternatively or in addition, the wrapper preferably incorporates no more than about 125 micrograms of the metal component of the sulphide scavenger compound per square centimetre of the wrapper, preferably no more than about 100 micrograms per square centimetre of the wrapper. Preferably the wrapper incorporates between about 0.25 micrograms and about 125 micrograms of the metal component of the sulphide scavenger compound per square centimetre of the wrapper, more preferably between about 2.5 micrograms and about 100 micrograms per square centimetre and more preferably between about 5 micrograms and about 100 micrograms per square centimetre, over the area of the wrapper into which the sulphide scavenger compound is incorporated. These preferred values are based on a wrapper having a basis weight of about 25 grams per square metre.

The wrapper of a single heat-not-burn article according to the invention preferably incorporates a total amount of the metal component of the sulphide scavenger compound of between about 10 micrograms and about 700 micrograms, more preferably between about 15 micrograms and about 500 micrograms.

Preferably, the sulphide scavenger compound is incorporated in a sufficient amount to achieve a reduction of at least about 30 percent by weight of hydrogen sulphide during a pre-heating test compared with an equivalent heat-not-burn article without the sulphide scavenger compound in the wrapper, more preferably at least about 50 percent, most preferably at least about 70 percent. For the purposes of such a comparison, both the heat-not-burn article with and without the sulphide scavenger compound in the wrapper are pre-heated in a pre-heating test as defined below.

In the pre-heating test, an heat-not-burn article is inserted into an aerosol-generating device comprising a heating element for heating the aerosol-forming substrate of the heat-not-burn article. The heating element is programmed to heat at 350 degrees Celsius for 30 seconds and then switch off, to simulate the pre-heating phase of the heat-not-burn article during normal use. During the heating of the heat-not-burn article, the heat-not-burn article is placed in a sealed glass vial so that the gas phase constituents released from the heat-not-burn article during heating are collected. A sample of the gas phase constituents collected within the vial is then removed and the concentration of hydrogen sulphide is determined using a liquid chromatography-mass spectrometry method. A suitable aerosol-generating device for the pre-heating test is the iQOS® heat-not-burn device from Philip Morris International, which is commercially available.

In preferred embodiments of the invention, the heat-not-burn article is adapted for use with an aerosol-generating device comprising a heating element. In such embodiments, the aerosol-forming substrate is preferably adapted to be penetrated by the heating element of an aerosol-generating device into which the heat-not-burn article is inserted during smoking. Where a front-plug is provided upstream of the aerosol-forming substrate, the front-plug may be adapted to be penetrated by the heating element.

In alternative embodiments of the invention, the heat-not-burn article may incorporate a heat source adjacent to the aerosol-forming substrate such that a separate aerosol-generating device is not required.

The aerosol-forming substrate of heat-not-burn articles according to the invention comprises tobacco. Preferably, the aerosol-forming substrate is a solid aerosol-forming substrate. The aerosol-forming substrate may comprise both solid and liquid components.

In a preferred embodiment, the aerosol-forming substrate comprises homogenised tobacco material. Preferably, the aerosol-forming substrate comprises a gathered sheet of homogenised tobacco material. As used herein, the term "homogenised tobacco material" denotes a material formed by agglomerating particulate tobacco.

Heat-not-burn articles according to the invention preferably further comprise a support element located immediately downstream of the aerosol-forming substrate so that the aerosol-forming substrate and the support element abut each other in an axial direction. The support element preferably prevents downstream movement of the aerosol-forming substrate when the upstream end of the heat-not-burn article is inserted into a device requiring insertion force, such as may be required when inserting the heat-not-burn article into a device having a heating element configured to penetrate the aerosol-forming substrate.

The aerosol-forming substrate is preferably located at the upstream end of the heat-not-burn article. Alternatively, a front-plug may be incorporated upstream of the aerosol-forming substrate.

Heat-not-burn articles according to the invention may further comprise an aerosol-cooling element located downstream of the support element. As used herein, the term "aerosol-cooling element" describes an element having a large surface area and a low resistance to draw. In use, an aerosol formed by volatile compounds released from the aerosol-forming substrate passes over and is cooled by the aerosol-cooling element before being inhaled by a user. In contrast to high resistance to draw filters and other mouthpieces, aerosol-cooling elements have a low resistance to draw. Chambers and cavities within an heat-not-burn article are also not considered to be aerosol-cooling elements.

Alternatively or in addition, heat-not-burn articles according to the invention may further comprise a mouthpiece located at the downstream end of the heat-not-burn article. The mouthpiece may comprise a filter. The filter may be formed from one or more suitable filtration materials. Many such filtration materials are known in the art. In one embodiment, the mouthpiece may comprise a filter formed from cellulose acetate tow.

Suitable aerosol-forming substrates, support elements, aerosol-cooling elements and mouthpieces are described in WO-A-2013/098405.

As set out above, the wrapper incorporating the sulphide scavenger compound circumscribes at least a portion of the heat-not-burn article. Preferably, the wrapper (or the region of the wrapper incorporating the sulphide scavenger compound) is provided around at least part of the portion of the heat-not-burn article that is adapted to be received into an heat-not-burn device. The sulphide scavenger compound is therefore provided in the region of the heat-not-burn article from which the hydrogen sulphide will be emitted. The portion of the heat-not-burn article that is adapted to be received into the heat-not-burn device typically includes the aerosol-forming substrate and the support element downstream of the aerosol-forming substrate, where present. Where present, the aerosol-cooling element may also be inserted into the heat-not-burn device. The mouthpiece, where present, will typically not be inserted into the heat-not-burn device.

Preferably, the wrapper or the portion of the wrapper incorporating the sulphide scavenger compound circumscribes at least the aerosol-forming substrate of the heat-not-burn article. With such an arrangement, the sulphide scavenger compound is provided in the immediate vicinity of the aerosol-forming substrate from which the hydrogen sulphide will be generated. Therefore, the sulphide scavenger compound is well positioned to come into contact with the hydrogen sulphide as it is released during the pre-heating phase such that it can be trapped or reacted into a more inert form before being detected by the consumer. Alternatively or in addition, the wrapper incorporating the sulphide scavenger compound may circumscribe the support element downstream of the aerosol-forming substrate, where present.

The wrapper incorporating the sulphide scavenger compound may circumscribe only a part of the heat-not-burn article, with the remainder of the heat-not-burn article circumscribed by one or more additional wrappers that do not contain a sulphide scavenger compound. Alternatively, the wrapper incorporating the sulphide scavenger compound may circumscribe the heat-not-burn article along substantially the full length.

In certain embodiments, the sulphide scavenger compound may be provided only in certain defined areas or portions of the wrapper. For example, where the sulphide scavenger compound is applied in the form of a coating layer, the coating layer may only be applied to certain areas of the wrapper, such as those that are proximate the aerosol-forming substrate. In other embodiments, the sulphide scavenger compound may be provided throughout substantially the entire wrapper.

The wrapper of the heat-not-burn article according to the present invention is formed of a sheet material incorporating the sulphide scavenger compound in some way, as described above. The wrapper is preferably a sheet of a fibrous material and particularly preferably, a sheet of paper.

Aerosol-generating systems according to the present invention comprise an heat-not-burn article as described in detail above in combination with an aerosol-generating device which is adapted to receive the upstream end of the heat-not-burn article during smoking. The aerosol-generating device comprises a heating element which is adapted to heat the aerosol-forming substrate in order to generate an aerosol during use. Preferably, the heating element is adapted to penetrate the aerosol-forming substrate when the heat-not-burn article is inserted into the aerosol-generating device.

Preferably, the aerosol-generating device additionally comprise a housing, an electrical power supply connected to the heating element and a control element configured to control the supply of power from the power supply to the heating element. It is this control element which controls the heating to produce the heating profile including the pre-heating phase discussed above.

Suitable aerosol-generating devices for use in the aerosol-generating system of the present invention are described in WO-A-2013/098405.

The present invention further extends to a method for the production of a heat-not-burn article as described above. The method comprises the steps of: providing an aerosol-forming substrate; combining the aerosol-forming substrate with one or more components to form a heat-not-burn article; and wrapping the heat-not-burn article with an outer wrapper comprising a metal-based sulphide scavenger compound, wherein the metal-based sulphide scavenger compound is a metal salt based on a transition metal. Such methods produce heat-not-burn articles with the sulphide scavenger compound in the outer wrapper.

In an alternative method according to the present invention, the method comprises the steps of: providing an aerosol-forming substrate circumscribed by a plug wrap comprising a metal-based sulphide scavenger compound, wherein the metal-based sulphide scavenger compound is a metal salt based on a transition metal; combining the aerosol-forming substrate with one or more components to form a heat-not-burn article; and wrapping the heat-not-burn article with an outer wrapper. Such methods produce heat-not-burn articles with the sulphide scavenger compound in the plug wrap of the aerosol-forming substrate.

Methods according to the invention can advantageously be carried out using existing apparatus and techniques for assembling heat-not-burn articles, since the sulphide scavenger compound can be incorporated into the appropriate wrapper prior to the assembly of the heat-not-burn article. The wrapping of the wrapper around the heat-not-burn article can then be carried out in a conventional manner.

The invention will now be further described, by way of example only, with reference to the accompanying drawings in which:
Figure 1 is a schematic view of a heat-not-burn article according to an embodiment of the invention, with the outer wrapper partially unwrapped; and
Figure 2 is a schematic cross-sectional view of the heat-not-burn article of Figure 1.

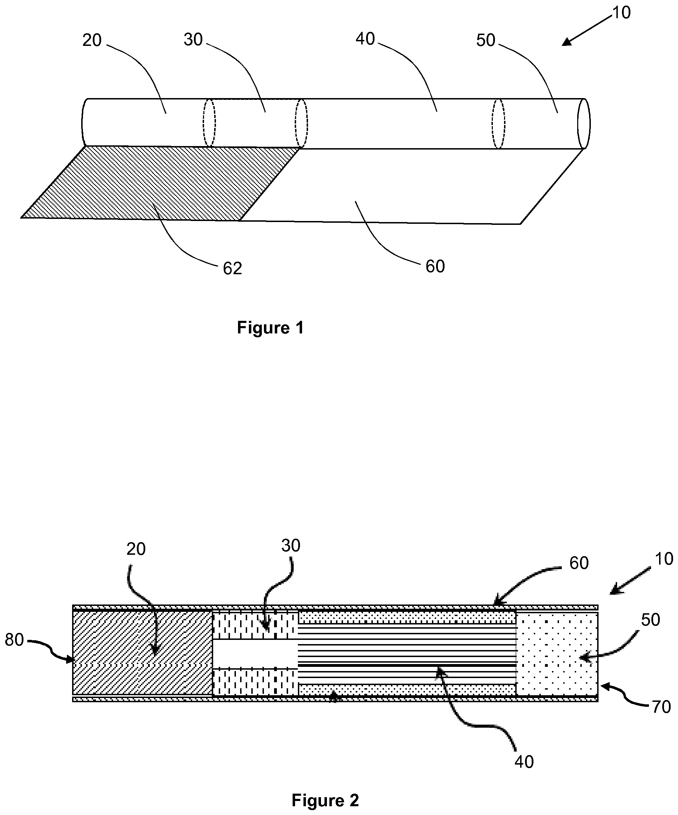

The heat-not-burn article 10 shown in Figures 1 and 2 comprises four elements arranged in coaxial alignment: an aerosol-forming substrate 20, a support element 30, an aerosol-cooling element 40, and a mouthpiece 50. Each of the four elements is circumscribed by a corresponding plug wrap (not shown). These four elements are arranged sequentially and are circumscribed by an outer wrapper 60 to form the heat-not-burn article 10. The heat-not-burn 10 has a proximal or mouth end 70, which a user inserts into his or her mouth during use, and a distal end 80 located at the opposite end of the heat-not-burn article 10 to the mouth end 70.

In use, air is drawn through the heat-not-burn article 10 by a user from the distal end 80 to the mouth end 70. The distal end 80 of the heat-not-burn article may also be described as the upstream end of the heat-not-burn article 10 and the mouth end 70 of the heat-not-burn article 10 may also be described as the downstream end of the heat-not-burn article 10. Elements of the heat-not-burn article 10 located between the mouth end 70 and the distal end 80 can be described as being upstream of the mouth end 70 or, alternatively, downstream of the distal end 80.

The aerosol-forming substrate 20 is located at the extreme distal or upstream end of the heat-not-burn article 10. In the embodiment illustrated in Figure 1, aerosol-forming substrate 20 comprises a gathered sheet of crimped homogenised tobacco material circumscribed by a wrapper. The crimped sheet of homogenised tobacco material comprises comprising glycerine as an aerosol-former.

The support element 30 is located immediately downstream of the aerosol-forming substrate 20 and abuts the aerosol-forming substrate 20. In the embodiment shown in Figure 1, the support element is a hollow cellulose acetate tube. The support element 30 locates the aerosol-forming substrate 20 at the extreme distal end 80 of the heat-not-burn article 10 so that it can be penetrated by a heating element of an aerosol-generating device. As described further below, the support element 30 acts to prevent the aerosol-forming substrate 20 from being forced downstream within the heat-not-burn article 10 towards the aerosol-cooling element 40 when a heating element of an heat-not-burn device is inserted into the aerosol-forming substrate 20. The support element 30 also acts as a spacer to space the aerosol-cooling element 40 of the heat-not-burn article 10 from the aerosol-forming substrate 20.

The aerosol-cooling element 40 is located immediately downstream of the support element 30 and abuts the support element 30. In use, volatile substances released from the aerosol-forming substrate 20 pass along the aerosol-cooling element 40 towards the mouth end 70 of the heat-not-burn article 10. The volatile substances may cool within the aerosol-cooling element 40 to form an aerosol that is inhaled by the user. In the embodiment illustrated in Figure 1, the aerosol-cooling element comprises a crimped and gathered sheet of polylactic acid circumscribed by a wrapper 90. The crimped and gathered sheet of polylactic acid defines a plurality of longitudinal channels that extend along the length of the aerosol-cooling element 40.

The mouthpiece 50 is located immediately downstream of the aerosol-cooling element 40 and abuts the aerosol-cooling element 40. In the embodiment illustrated in Figure 1, the mouthpiece 50 comprises a conventional cellulose acetate tow filter of low filtration efficiency.

The outer wrapper 60 is a sheet of cigarette paper which has been impregnated with a solution of a sulphide scavenger compound in the region 62 of the outer wrapper (shaded in Figure 1) overlying the aerosol-forming substrate 20 and the support element 30. Examples of suitable sulphide scavenger compounds are provided in Table 1 below.

To assemble the heat-not-burn article 10, the four elements described above are aligned and tightly wrapped within the outer wrapper 60. An optional row of perforations is provided in a region of the outer wrapper 60 circumscribing the support element 30 of the heat-not-burn article 10.

In the embodiment illustrated in Figure 1, a distal end portion of the outer wrapper 60 of the heat-not-burn article 10 is circumscribed by a band of tipping paper (not shown).

The heat-not-burn article 10 illustrated in Figure 1 is designed to engage with an aerosol-generating device comprising a heating element in order to be smoked or consumed by a user. In use, the heating element of the aerosol-generating device heats the aerosol-forming substrate 20 of the heat-not-burn article 10 to a sufficient temperature to form an aerosol, which is drawn downstream through the heat-not-burn article 10 and inhaled by the user.

During the pre-heating phase, the sulphide scavenger compound in the outer wrapper 60 acts to reduce the level of hydrogen sulphide emitted from the aerosol-forming substrate. In a pre-heating test as defined above, the reduction achieved is at least 30 percent compared to an heat-not-burn article of a similar construction but with an outer wrapper formed from a conventional cigarette paper. As demonstrated in the examples below, for many sulphide scavenger compounds, a reduction of up to 70 percent in the level of hydrogen sulphide can be achieved. Such a reduction in the level of hydrogen sulphide means that the malodour from the hydrogen sulphide is minimised and may not be detectable by the consumer at all.

In the specific embodiment described above, the sulphide scavenger compound is incorporated into the outer wrapper of the heat-not-burn article. However, the skilled person would appreciate that the sulphide scavenger compound could additionally or alternatively be incorporated into one or more of the plug wraps circumscribing the individual elements.

### Examples

For each of the sulphide scavenger compounds shown below in Table 1, an heat-not-burn article according to the invention (as described above with reference to the figures) was produced, with 0.1 millilitres of a solution of the sulphide scavenger compound applied to the region 62 of the outer wrapper using a micropipette and spread evenly around the circumference of the heat-not-burn article. Each heat-not-burn article was subjected to the pre-heating test defined above.

The percentage reductions in hydrogen sulphide and methanethiol were measured relative to a control sample in which the solvent without the sulphide scavenger compound was applied in the same way to an heat-not-burn article of the same construction.

For each sulphide scavenger compound, the reduction shown is an average of the reductions measured over four samples.

It can be seen from the results below that for each sulphide scavenger compound, a reduction of approximately 70 percent of hydrogen sulphide was observed relative to the control sample. Significant reductions in methanethiol were also observed, in particular for the copper based scavenger compounds.

**Table 1**

| **Sulphide scavenger compound** | **Amount of transition metal component** (micrograms per square centimetre) | **% Reduction in Hydrogen Sulphide** | **% Reduction in Methanethiol** |
|---|---|---|---|
| Zinc chloride | 400 | 73 | 43 |
| Zinc chloride | 100 | 70 | 28 |
| Zinc chloride | 25 | 67 | 22 |
| Copper (II) Sulphate pentahydrate | 100 | 74 | 71 |
| Copper (II) Sulphate pentahydrate | 25 | 71 | 69 |
| Copper (II) Sulphate pentahydrate | 6.25 | 68 | 64 |

## Claims

1. A wrapper (60) for a heat-not-burn article (10) comprising an aerosol-forming tobacco substrate (20), the wrapper (60) comprising a sulphide scavenger compound, wherein the sulphide scavenger compound is a metal salt, wherein the metal salt is a carbonate, chloride, sulphate, hydroxide, nitrate, malate, acetate, citrate or bromide, and wherein the sulphide scavenger compound is based on a transition metal.

2. A heat-not-burn article (10) comprising:
an aerosol-forming tobacco substrate (20) ; and
a wrapper (60) according to claim 1 circumscribing at least a part of the heat-not-burn article (10).

3. A heat-not-burn article (10) according to claim 2 wherein the sulphide scavenger compound is based on zinc or copper.

4. A heat-not-burn article (10) according to claim 2 or 3 wherein the wrapper (60) comprises the sulphide scavenger compound combined with a polymeric binder.

5. A heat-not-burn article (10) according to any one of claims 2 to 4 wherein an outer layer incorporating the sulphide scavenger compound is provided on at least one surface of the wrapper (60).

6. A heat-not-burn article (10) according to any one of claims 2 to 5 the wrapper (60) is formed of a sheet of a fibrous material and wherein the sulphide scavenger compound is dispersed within the fibrous material.

7. A heat-not-burn article (10) according to any one of claims 2 to 6 wherein the sulphide scavenger compound is impregnated into the wrapper (60).

8. A heat-not-burn article (10) according to any one of claims 2 to 7 wherein the amount of the metal component of the sulphide scavenger compound in the wrapper (60) is between 0.01 percent and 5 percent by weight based on the total weight of the wrapper (60) and the sulphide scavenger compound.

9. A heat-not-burn article (10) according to any one of claims 2 to 8 wherein the amount of the metal component of the sulphide scavenger compound in the wrapper (60) is between 0.25 micrograms per square centimetre and 125 micrograms per square centimetre of the wrapper (60).

10. A heat-not-burn article (10) according to any one of claims 2 to 9 wherein the sulphide scavenger compound in the wrapper (60) provides a reduction of at least 50 percent by weight of hydrogen sulphide during a pre-heating test compared with an equivalent heat-not-burn article without the sulphide scavenger compound in the wrapper (60), wherein in the pre-heating test a heating element for heating the aerosol-forming substrate in the heat-not-burn article is programmed to heat at 350 degrees Celsius for 30 seconds and then switch off.

11. A heat-not-burn article (10) according to any one of claims 2 to 10 wherein the wrapper (60) comprising the sulphide scavenger compound is the outer wrapper (60).

12. An aerosol-generating system comprising:
an aerosol-generating device comprising a heating element; and
a heat-not-burn article (10) for use with the aerosol-generating device, the heat-not-burn article comprising:
an aerosol-forming tobacco substrate (20); and
a wrapper (60) according to claim 1 circumscribing at least a part of the heat-not-burn article (10).

13. A method for the production of a heat-not-burn article (10), the method comprising the steps of:
providing an aerosol-forming tobacco substrate (20);
combining the aerosol-forming tobacco substrate (20) with one or more components to form the heat-not-burn article (10); and
wrapping the heat-not-burn article (10) with an outer wrapper (60), wherein the outer wrapper is a wrapper according to claim 1.

14. Use of a sulphide scavenger compound based on a transition metal salt in the wrapper (60) of a heat-not-burn article (10) to reduce the level of hydrogen sulphide released during heating of the heat-not-burn article (10).

## Patentansprüche

1. Umhüllung (60) für einen Erwärmen-nicht-Verbrennen-Artikel (10), aufweisend ein aerosolbildendes Tabaksubstrat (20), wobei die Umhüllung (60) eine Sulfidfängerverbindung aufweist, wobei die Sulfidfängerverbindung ein Metallsalz ist, wobei das Metallsalz ein Carbonat, Chlorid, Sulfat, Hydroxid, Nitrat, Malat, Acetat, Citrat oder Bromid ist, und wobei die Sulfidfängerverbindung auf einem Übergangsmetall basiert.

2. Erwärmen-nicht-Verbrennen-Artikel (10), aufweisend:
ein aerosolbildendes Tabaksubstrat (20); und
eine Umhüllung (60) nach Anspruch 1, die mindestens einen Teil des Erwärmen-nicht-Verbrennen-Artikels (10) abgrenzt.

3. Erwärmen-nicht-Verbrennen-Artikel (10) nach Anspruch 2, wobei die Sulfidfängerverbindung auf Zink oder Kupfer basiert.

4. Erwärmen-nicht-Verbrennen-Artikel (10) nach Anspruch 2 oder 3, wobei die Umhüllung (60) die Sulfidfängerverbindung in Kombination mit einem polymeren Bindemittel aufweist.

5. Erwärmen-nicht-Verbrennen-Artikel (10) nach einem der Ansprüche 2 bis 4, wobei eine äußere Schicht, welche die Sulfidfängerverbindung einschließt, auf mindestens einer Fläche der Umhüllung (60) vorgesehen ist.

6. Erwärmen-nicht-Verbrennen-Artikel (10) nach einem der Ansprüche 2 bis 5, wobei die Umhüllung (60) aus einem Flächengebilde eines Fasermaterials gebildet ist, und wobei die Sulfidfängerverbindung in dem Fasermaterial dispergiert ist.

7. Erwärmen-nicht-Verbrennen-Artikel (10) nach einem der Ansprüche 2 bis 6, wobei die Sulfidfängerverbindung in die Umhüllung (60) imprägniert ist.

8. Erwärmen-nicht-Verbrennen-Artikel (10) nach einem der Ansprüche 2 bis 7, wobei die Menge der Metallkomponente der Sulfidfängerverbindung in der Umhüllung (60) basierend auf dem Gesamtgewicht der Umhüllung (60) und der Sulfidfängerverbindung zwischen 0,01 Gew.-% und 5 Gew.-% beträgt.

9. Erwärmen-nicht-Verbrennen-Artikel (10) nach einem der Ansprüche 2 bis 8, wobei die Menge der Metallkomponente der Sulfidfängerverbindung in der Umhüllung (60) zwischen 0,25 Mikrogramm pro Quadratzentimeter und 125 Mikrogramm pro Quadratzentimeter der Umhüllung (60) beträgt.

10. Erwärmen-nicht-Verbrennen-Artikel (10) nach einem der Ansprüche 2 bis 9, wobei die Sulfidfängerverbindung in der Hülle (60) verglichen mit einem äquivalenten Erwärmen-nicht-Verbrennen-Artikel ohne die Sulfidfängerverbindung in der Umhüllung (60) eine Reduktion von mindestens 50 Gewichtsprozent Schwefelwasserstoff während eines Vorwärmtests bereitstellt, wobei in dem Vorwärmtest ein Heizelement zum Erwärmen des aerosolbildenden Substrats in dem Erwärmen-nicht-Verbrennen-Artikel derart programmiert ist, dass es 30 Sekunden lang bei 350 Grad Celsius erwärmt und dann abschaltet.

11. Erwärmen-nicht-Verbrennen-Artikel (10) nach einem der Ansprüche 2 bis 10, wobei die Umhüllung (60), welche die Sulfidfängerverbindung aufweist, die äußere Umhüllung (60) ist.

12. Aerosolerzeugungssystem, aufweisend:
eine Aerosolerzeugungsvorrichtung, die ein Heizelement aufweist; und
einen Artikel (10) zum Gebrauch mit der Aerosolerzeugungsvorrichtung, wobei der Artikel zum Gebrauch mit der Aerosolerzeugungsvorrichtung aufweist:
ein aerosolbildendes Tabaksubstrat (20); und
eine Umhüllung (60) nach Anspruch 1, die mindestens einen Teil des Erwärmen-nicht-Verbrennen-Artikels (10) abgrenzt.

13. Verfahren zur Herstellung eines Erwärmen-nicht-Verbrennen-Artikels (10), wobei das Verfahren die Schritte aufweist:
Bereitstellen eines aerosolbildenden Tabaksubstrats (20);
Kombinieren des aerosolbildenden Tabaksubstrats (20) mit einer oder mehreren Komponenten, um den Erwärmen-nicht-Verbrennen-Artikel (10) zu bilden; und
Umhüllen des Erwärmen-nicht-Verbrennen-Artikels (10) mit einer äußeren Umhüllung (60), wobei die äußere Umhüllung eine Umhüllung nach Anspruch 1 ist.

14. Gebrauch einer Sulfidfängerverbindung basierend auf einem Übergangsmetallsalz in der Umhüllung (60) eines Erwärmen-nicht-Verbrennen-Artikels (10), um den Gehalt an Wasserstoffsulfid zu reduzieren, das während des Erwärmens des Erwärmen-nicht-Verbrennen-Artikels (10) freigegeben wird.

## Revendications

1. Enveloppe (60) pour un article chauffé non brûlé (10) comprenant un substrat de tabac formant aérosol (20), l'enveloppe (60) comprenant un composé piégeur de sulfure, dans laquelle le composé piégeur de sulfure est un sel métallique, dans laquelle le sel métallique est un carbonate, chlorure, sulfate, hydroxyde, nitrate, malate, acétate, citrate ou bromure, et dans laquelle le composé piégeur de sulfure est basé sur un métal de transition.

2. Article chauffé non brûlé (10) comprenant :
un substrat de tabac formant aérosol (20) ; et
une enveloppe (60) selon la revendication 1 entourant au moins une partie de l'article chauffé non brûlé (10).

3. Article chauffé non brûlé (10) selon la revendication 2, dans lequel le composé piégeur de sulfure est à base de zinc ou de cuivre.

4. Article chauffé non brûlé (10) selon la revendication 2 ou 3, dans lequel l'enveloppe (60) comprend le composé piégeur de sulfure combiné avec un liant polymère.

5. Article chauffé non brûlé (10) selon l'une quelconque des revendications 2 à 4, dans lequel une couche externe incorporant le composé piégeur de sulfure est fournie sur au moins une surface de l'enveloppe (60).

6. Article chauffé non brûlé (10) selon l'une quelconque des revendications 2 à 5, l'enveloppe (60) est formée d'une feuille d'un matériau fibreux et dans lequel le composé piégeur de sulfure est dispersé dans le matériau fibreux.

7. Article chauffé non brûlé (10) selon l'une quelconque des revendications 2 à 6, dans lequel le composé piégeur de sulfure est imprégné dans l'enveloppe (60).

8. Article chauffé non brûlé (10) selon l'une quelconque des revendications 2 à 7, dans lequel la quantité du composant métallique du composé piégeur de sulfure dans l'enveloppe (60) est comprise entre 0,01 pour cent et 5 pour cent en poids sur la base du poids total de l'enveloppe (60) et du composé piégeur de sulfure.

9. Article chauffé non brûlé (10) selon l'une quelconque des revendications 2 à 8, dans lequel la quantité de composant métallique du composé piégeur de sulfure dans l'enveloppe (60) est comprise entre 0,25 microgrammes par centimètre carré et 125 microgrammes par centimètre carré de l'enveloppe (60).

10. Article chauffé non brûlé (10) selon l'une quelconque des revendications 2 à 9, dans lequel le composé piégeur de sulfure dans l'enveloppe (60) fournit une réduction d'au moins 50 pour cent en poids de sulfure d'hydrogène pendant un essai de préchauffage comparé avec un article chauffé non brûlé équivalent sans le composé piégeur de sulfure dans l'enveloppe (60), dans lequel dans le test de préchauffage un élément de chauffage pour chauffer le substrat formant aérosol dans l'article chauffé non brûlé est programmé pour se chauffer à 350 degrés Celsius pendant 30 secondes, puis, il s'éteint.

11. Article chauffé non brûlé (10) selon l'une quelconque des revendications 2 à 10, dans lequel l'enveloppe (60) comprenant le composé piégeur de sulfure est l'enveloppe extérieure (60).

12. Système générateur d'aérosol comprenant :
un dispositif générateur d'aérosol comprenant un élément de chauffage ; et
un article chauffé non brûlé (10) pour une utilisation avec le dispositif de génération d'aérosol, l'article chauffé non brûlé comprenant :
un substrat de tabac formant aérosol (20) ; et
une enveloppe (60) selon la revendication 1 entourant au moins une partie de l'article chauffé non brûlé (10).

13. Procédé pour la production d'un article chauffé non brûlé (10), le procédé comprenant les étapes :
de fourniture d'un substrat de tabac formant aérosol (20) ;
de combination du substrat de tabac formant aérosol (20) avec un ou plusieurs composants pour former l'article chauffé non brûlé (10) ; et
d'enveloppement de l'article chauffé non brûlé (10) avec une enveloppe extérieure (60), dans lequel l'enveloppe extérieure est une enveloppe selon la revendication 1.

14. Utilisation d'un composé piégeur de sulfure à base d'un sel de métal de transition dans l'enveloppe (60) d'un article chauffé non brûlé (10) pour réduire le niveau de sulfure d'hydrogène libéré pendant le chauffage de l'article chauffé non brûlé (10).
